# EUROPEAN PATENT SPECIFICATION

(11) **EP 2 381 847 B1**
(45) Date of publication and mention of the grant of the patent: **14.01.2015**
(21) Application number: 09756349.8
(22) Date of filing: 11.11.2009
(51) Int. Cl.: A61B 5/151

(54) **SKIN PRICKING DEVICE**
HAUTPUNKTIONSVORRICHTUNG
DISPOSITIF DE PIQÛRE DE LA PEAU

(30) Priority: 17.11.2008 GB 0820969
(43) Date of publication of application: 02.11.2011
(73) Proprietor: OWEN MUMFORD LIMITED, Oxford, Oxfordshire OX20 ITU (GB)
(72) Inventor: NICHOLLS, Clive, Great Haseley Oxfordshire OX44 7JJ (GB); HUDSON, Christopher, William, Witney Oxfordshire OX29 4PP (GB)
(74) Representative: Lind, Robert
(86) International application number: PCT/GB2009/051510
(87) International publication number: WO 2010/055330

(56) References cited:
- WO-A-2005/013825
- WO-A-2008/009984
- GB-A- 2 440 119
- US-A- 4 449 529
- US-A- 6 136 013
- US-A1- 2002 004 196
- US-A1- 2007 225 742

## Description

### Field of the Invention

The present invention relates to a skin pricking device and in particular, though not necessarily, to a skin pricking device for use in providing a sample of blood.

### Background

In the medical and related diagnostic and testing fields, it is often required to take small samples of blood from a subject for the purpose of testing or analysing the blood. A common way of achieving this is by using a small needle, or lancet, to pierce the skin at a location where blood vessels are close to the surface. In order to avoid infection and contamination, lancing devices are preferably intended for single use and are disposable. They must therefore be compact and cheap to manufacture.

A number of disposable lancing devices are currently on the market. These include the Unistik™ manufactured and marketed by Owen Mumford Ltd (Woodstock, UK). The current designs comprise a moulded plastics casing within which is mounted a short needle attached to a spring. A trigger is formed in the casing which, when depressed, releases the spring causing the needle to be fired out through an opening in the casing. Some of the current designs require an operator to preload or cock the spring prior to firing. In other designs, the lancing devices are supplied already cocked. It is also generally necessary for users to remove a cap from the front of the device or the needle prior to firing. Users must therefore perform at least two steps, and sometimes three, in order to perform the blood sampling procedure. Furthermore, these designs comprise several components that must be individually fabricated and assembled together, increasing the complexity and cost of manufacture.

US2007/0225742 describes another known disposable lancing device which dispenses with the removal of a resin cover encapsulating a pricking element. A lancet structure is inserted within a lancet holder and a force which separates a lancet cover encapsulating the tip of the pricking element form a lancet body thereby exposing, within the lancet holder, the sharp tip portion of the pricking element. The lancet and ejector mechanism are separately formed so that they can be easily combined and integrated with one another.

There exists a desire for a lancing device that is more straightforward to manufacture than current designs. Of course, any improved design must be reliable, ensuring that the device is provided to users in an operable condition, and must be simple to use. The present invention has been devised with the foregoing in mind.

### Summary

According to the present invention there is provided a skin pricking device and a method as set out in the accompanying claims. Further herein disclosed is a device comprises a housing, a lancet formed integrally with a spring and contained within the housing, and a trigger for holding the spring in a compressed state until actuation of the trigger by a user releases the spring. The spring can be a folded flat spring, and the spring and lancet can be formed from a single strip of material. The trigger may comprise a thumb plate and formations depending from the thumb plate for cooperating with formations on the spring. The trigger may be formed integrally with the housing. Alternatively, the trigger may be formed integrally with the spring.

The housing may have an opening for loading the spring into the housing and has an opening for accessing the trigger when the spring is loaded into the housing. When the spring is loaded into the housing the trigger may fit into the trigger opening such that the spring is retained within the housing. The housing may also have an opening through which the lancet can exit the housing when the spring extends.

Further herein disclosed is a skin pricking device. The device comprises a housing, and, contained within the housing, a lancet, a spring for driving the lancet, and a trigger for holding the spring in a compressed state until actuation of the trigger by a user releases the spring, at least the spring and the trigger being formed integrally with one another. The lancet may be formed integrally with the spring.

Further herein disclosed is a method of manufacturing a skin pricking device, the skin pricking device comprising a housing and a lancet mechanism, the lancet mechanism comprising a lancet, a spring for driving the lancet, the lancet being disposed at a distal end of the spring, and a trigger, at least the spring and the trigger being formed integrally with one another. The method comprises advancing the lancet mechanism into the housing through a loading opening in the housing, and blocking further movement of the distal end of the spring whilst applying a force to a proximal end of the spring to compress the spring until the spring is latched by the trigger to lock the spring in a compressed state. The force may also move the trigger further into the housing to a position where cooperating formations of the trigger and spring are engaged.

The further movement of the distal end of the spring may be prevented by a blocking element temporarily located adjacent to a lancet exit opening in the housing through which the lancet projects when the device is fired.

### Brief Description of the Drawings

Figure 1 is a perspective view of a skin pricking device according to an embodiment of the present invention;
Figure 2 is a cross-section through the skin pricking device of Figure 1 in a vertical plane;
Figure 3 is a perspective view of the housing of the skin pricking device of Figure 1;
Figure 4 is a cross-section through the housing of Figure 2 in a vertical plane;

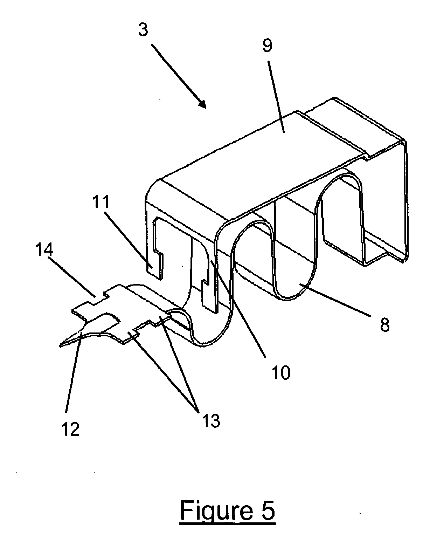

Figure 5 is a perspective view of the driver unit of the skin pricking device of Figure 1, prior to loading of the spring portion; and
Figure 6 is a perspective view of the driver unit of the skin pricking device of Figure 1, with the spring portion loaded and with the lancet cap covering the lancet.

### Detailed Description

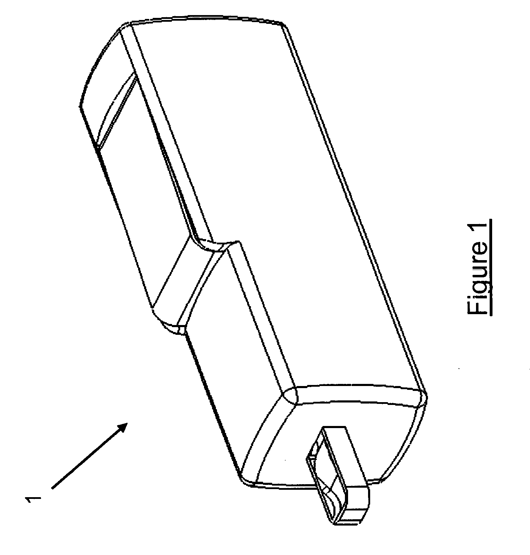

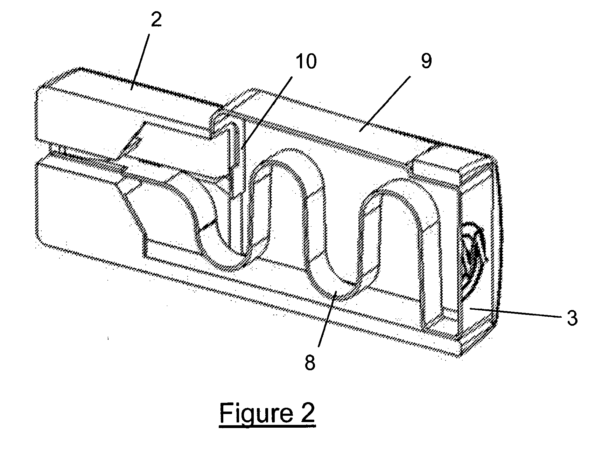

There is illustrated in Figure 1 a skin pricking device designed for pricking a person's skin, for example to provide a small sample of blood. Typically the device is used to prick the pad of a person's finger, leaving a small spot of blood on the finger. This spot can then be collected, e.g. using a test strip, for use in performing a measurement or test. The skin pricking device 1 has a housing 2 and a lancet mechanism 3 loaded into the housing as illustrated in Figure 2.

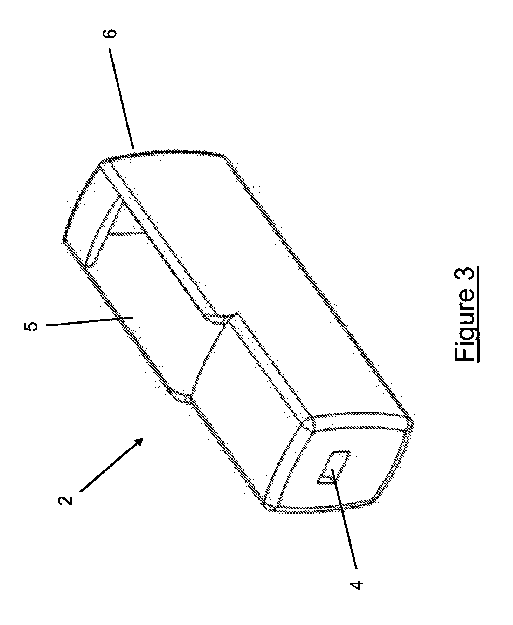

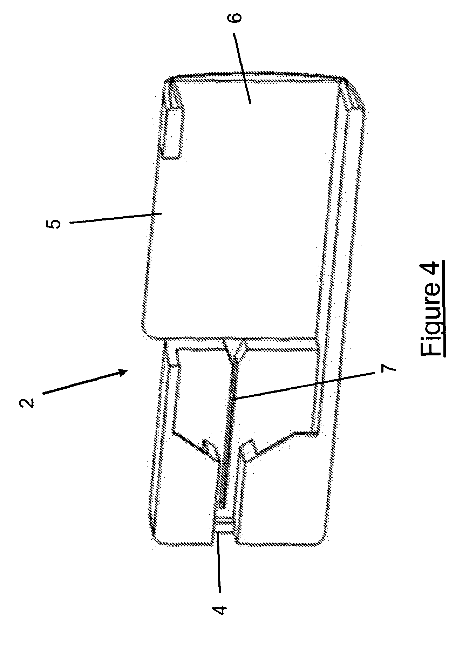

Figures 3 and 4 show the housing 2 and lancet mechanism 3 respectively. The housing 2 comprises a single, moulded piece in which there are formed an aperture 4 out of which a lancet can protrude, an opening for a trigger 5, and a mouth 6 for loading the driver and lancet unit 3 into the housing 2. Formed integrally within the inner walls of the housing are a pair of guide tracks 7 (see Figure 4). These guide tracks 7 extend longitudinally along respective side walls of the housing, from an edge substantially adjacent to the aperture 4 to a point about one third of the way along the length of the housing.

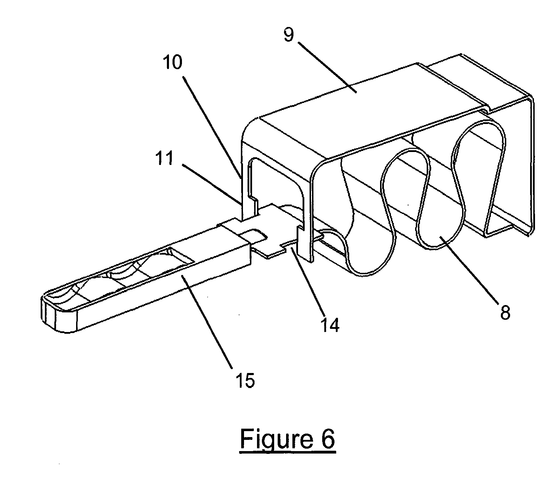

The lancet mechanism 3 is formed from a single strip of metal that is moulded, stamped, pressed or cut to an appropriate shape. The strip is folded to form an overall u-shaped piece, one side of which is further folded to form a flat folded spring portion 8, and the opposite side of which is formed into a trigger portion 9. The distal end of the spring portion 8 is formed with a lancet 12 and each side of the spring portion 8 immediately adjacent to the lancet 12 is formed with a pair of latch protrusions 13, each pair of latch protrusions 13 defining a latch recess 14 between them. The distal end of the trigger portion 9 is formed with two prongs or arms 10 that extend perpendicularly from the trigger portion 9 towards the spring portion 8. The end of each arm 10 is also formed with a stop or keeper 11, with each stop 11 protruding from its respective arm towards the other arm so as to narrow the space between the arms. These stops 11 can engage with the latch recesses 14 disposed on the end of spring portion 8, holding it in a compressed configuration, as shown in Figure 6. The lancet mechanism 3 is also provided with a plastic lancet cap 15 that covers the lancet 12 as shown in Figure 6, and is of sufficient length to extend from the lancet 12 out through the lancet aperture 4 when the device 1 is assembled and loaded. The lancet cap 15 will typically be moulded onto the lancet 12 prior to assembly and is only removed immediately prior to use.

The device 1 is assembled on an assembly rig by loading the lancet end of the lancet mechanism 3 into the mouth 6 of the housing 2, and pushing the lancet mechanism 3 into the housing 2. The size of the mouth 6 of the housing 2 is slightly smaller than the distance between the trigger portion 9 and the furthest parts of the spring portion 8 such that the trigger portion 9 flexes towards the spring portion 8 as it enters the mouth 6 of the housing 2. This flexing of the trigger portion 9 causes the stops 11 on the ends of the trigger arms 10 to move towards the spring portion 8 such that the stops 11 move beyond the plane in which the guide tracks 7 lie. As the lancet mechanism 3 moves further into the housing 2 the latch protrusions 13 move into the guide tracks 7 and the lancet cap 15 moves towards the lancet aperture 4. The lancet cap 15 is prevented from fully exiting the housing 2 by means of a blocking element on the assembly rig (not shown). The additional length provided by the lancet cap 15 causes the distal end of the lancet cap 15 to reach the blocking element before the lancet and driver unit 3 is fully loaded into the housing 2, such that the force applied to push the lancet mechanism 3 into the housing 2 causes the spring portion 8 to compress.

The compression of the spring portion 8 causes the rearmost of each pair of latch protrusions to pass through the space between the arms 10, that space being wider than the space between the stops 11. Once the lancet mechanism 3 has moved far enough into the housing 2 such that the tops of trigger arms 10 abut the edge of the trigger opening 5, the trigger portion 9 will slot into the trigger opening 5 causing the trigger portion 9 to straighten and the trigger arms 10 to move away from the spring portion 8 back into their original position. This movement allows the stops 11 to move into the latch recesses 14 such that, when the lancet aperture 4 is uncovered, the spring portion 8 will partially unload until the rearmost of each pair of latch protrusions 13 meets with the stops 11 preventing any further unloading. This partial unloading allows the distal end of the lancet cap 15 to further exit the housing 2 but maintains the spring portion 8 in a loaded configuration. Once the device 1 has been fully assembled it is then irradiated in order to sterilise it ready for use. Alternatively, the lancet mechanism could be sterilized prior to assembly.

In use, a user first removes the lancet cap 15 by gripping the distal end that has exited the housing 2 through the lancet aperture 4. He or she then places the device 1 against the skin, and releases the spring portion 8 by pressing the trigger portion 9 into the device. The pressing of the trigger portion 9 causes it to flex and the stops 11 on the ends of the trigger arms 10 move towards the spring portion 8. The stops 11 then move beyond the latch recesses 14, freeing the spring portion 8 to unload from its compressed configuration and extend. The force provided by the unloading of the spring portion 8 causes the spring portion to over extend such that the lancet 12 moves out through the lancet aperture 4 of the housing 2, striking the skin. The spring portion then contracts slightly, taking the lancet 12 back inside the housing 2.

The guide tracks 7 on the interior of the housing 2 provide that, when the device is fired and the spring portion 8 unloads, the latch protrusions 13 on the lancet end of the lancet mechanism 3 move through these guide tracks 7, thus ensuring that the lancet 12 moves through the lancet aperture 4 without making contact with the housing 2.

The single component lancet mechanism should be formed from a suitably resilient material, such as a metal or plastic" to enable it to act as a spring and in order to allow the trigger portion to flex into the housing when actuating the device. The material should also be capable of being shaped or moulded as required.

In an alternative embodiment, the trigger portion, including the trigger arms and stops, can be formed with the housing, and separately to the rest of the lancet mechanism. The device could then be assembled by pushing the lancet mechanism into the housing until the latch protrusions meet the stops. The device can then be operated as described above. In such an embodiment the housing should be formed from a suitably resilient material, such as a plastic or metal, in order to allow the trigger portion to flex into the housing when actuating the device whilst also being sufficiently rigid to maintain the overall shape of the housing. The material should also be capable of being shaped or moulded as required.

The device described provides a reliable lancing device, requiring no more than two steps to use, and whose manufacture and assembly is simple, also requiring a minimum number of steps. This reduces manufacturing costs and assembly times, which is especially important for high volume manufacturing.

It will be appreciated by the person of skill in the art that various modifications may be made to the above-described embodiments without departing from the scope of the present invention.

## Claims

1. A skin pricking device (1) comprising:
a housing (2);
a lancet needle (12) formed integrally with a spring (8) and contained within the housing (2);
a trigger (9) for holding the spring (8) in a compressed state until actuation of the trigger (9) by a user releases the spring (8);
wherein the trigger (9) is formed integrally with the spring (8); and
wherein the housing (2) has an opening (5) for accessing the trigger (9) when the spring (8) is loaded into the housing (2).

2. A skin pricking device (1) as claimed in claim 1, wherein the spring (8) is a folded flat spring.

3. A skin pricking device (1) as claimed in any preceding claim, wherein the spring (8) and lancet needle (12) are formed from a single strip of material.

4. A skin pricking device (1) as claimed in any preceding claim, wherein the trigger (9) comprises a thumb plate and formations depending from the thumb plate for cooperating with formations on the spring (8) to hold the spring (8) in a compressed state.

5. A skin pricking device (1) as claimed in any preceding claim, wherein the housing (2) has an opening (6) for loading the spring (8) into the housing (2).

6. A skin pricking device (1) as claimed in claim 1, wherein when the spring (8) is loaded into the housing (2) the trigger (9) fits into the trigger (9) opening such that the spring (8) is retained within the housing (2).

7. A skin pricking device (1) as claimed in any preceding claim, wherein the housing (2) has an opening (4) for allowing the lancet needle (12) to exit the housing (2) when the spring (8) extends.

8. A method of manufacturing a skin pricking device (1), the skin pricking device (1) comprising a housing (2) and a lancet mechanism (3), the lancet mechanism (3) comprising a lancet needle (12), a spring (8) for driving the lancet, the lancet needle (12) being disposed at a distal end of the spring (8), and a trigger (9), at least the spring (8) and the trigger (9) being formed integrally with one another, and wherein the housing (2) has an opening (5) for accessing the trigger (9) when the spring (8) is loaded into the housing (2) the method comprising:
loading the lancet mechanism (3) into the housing (2); and
preventing further movement of the distal end of the spring (8) whilst applying a force to a proximal end of the spring (8) to compress the spring (8) until the spring (8) is latched by the trigger (9) to lock the spring (8) in a compressed state.

9. A method as claimed in claim 8, wherein the force also moves the trigger (9) further into the housing to a position where cooperating formations of the trigger (9) and spring (8) are engaged.

10. A method as claimed in any of claims 8 or 9, wherein said further movement of the distal end of the spring (8) is prevented by a blocking element temporarily located adjacent to a lancet needle exit opening (4) in the housing (2) through which the lancet needle (12) projects when the device (1) is fired.

## Patentansprüche

1. Hautpunktionsvorrichtung (1), die Folgendes umfasst:
ein Gehäuse (2),
eine Lanzettnadel (12), die integral mit einer Feder (8) geformt und innerhalb des Gehäuses (2) enthalten ist,
einen Auslöser (9) zum Halten der Feder (8) in einem zusammengedrückten Zustand, bis eine Betätigung des Auslösers (9) durch einen Benutzer die Feder (8) freigibt,
wobei der Auslöser (9) integral mit der Feder (8) geformt ist und
wobei das Gehäuse (2) eine Öffnung (5) zum Zugreifen auf den Auslöser (9), wenn die Feder (8) in das Gehäuse (2) geladen ist, hat.

2. Hautpunktionsvorrichtung (1) nach Anspruch 1, wobei die Feder (8) eine gefaltete Blattfeder ist.

3. Hautpunktionsvorrichtung (1) nach einem der vorhergehenden Ansprüche, wobei die Feder (8) und die Lanzettnadel (12) aus einem einzigen Streifen Material geformt sind.

4. Hautpunktionsvorrichtung (1) nach einem der vorhergehenden Ansprüche, wobei der Auslöser (9) eine Daumenplatte und an der Daumenplatte hängende Formationen umfasst, um mit Formationen an der Feder (8) zusammenzuwirken, um die Feder (8) in einem zusammengedrückten Zustand zu halten.

5. Hautpunktionsvorrichtung (1) nach einem der vorhergehenden Ansprüche, wobei das Gehäuse (2) eine Öffnung (6) zum Laden der Feder (8) in das Gehäuse (2) hat.

6. Hautpunktionsvorrichtung (1) nach Anspruch 1, wobei, wenn die Feder (8) in das Gehäuse (2) geladen ist, der Auslöser (9) derart in die Auslöser- (9) Öffnung passt, dass die Feder (8) innerhalb des Gehäuses (2) zurückgehalten wird.

7. Hautpunktionsvorrichtung (1) nach einem der vorhergehenden Ansprüche, wobei, wenn sich die Feder (8) ausdehnt, das Gehäuse (2) eine Öffnung (4) hat, um zu ermöglichen, dass die Lanzettnadel (12) aus dem Gehäuse (2) austritt.

8. Verfahren zum Herstellen einer Hautpunktionsvorrichtung (1), wobei die Hautpunktionsvorrichtung (1) ein Gehäuse (2) und einen Lanzettenmechanismus (3) umfasst, wobei der Lanzettenmechanismus (3) eine Lanzettnadel (12), eine Feder (8) zum Antreiben der Lanzette, wobei die Lanzettnadel (12) an einem distalen Ende der Feder (8) angeordnet ist, und einen Auslöser (9) umfasst, wobei wenigstens die Feder (8) und der Auslöser (9) integral miteinander geformt sind und wobei das Gehäuse (2) eine Öffnung (5) zum Zugreifen auf den Auslöser (9), wenn die Feder (8) in das Gehäuse (2) geladen ist, hat, wobei das Verfahren Folgendes umfasst:
das Laden des Lanzettenmechanismus (3) in das Gehäuse (2) und
das Verhindern einer weiteren Bewegung des distalen Endes der Feder (8), während eine Kraft auf ein proximales Ende der Feder (8) ausgeübt wird, um die Feder (8) zusammenzudrücken, bis die Feder (8) durch den Auslöser (9) eingeklinkt wird, um die Feder (8) in einem zusammengedrückten Zustand zu arretieren.

9. Verfahren nach Anspruch 8, wobei die Kraft ebenfalls den Auslöser (9) weiter in das Gehäuse zu einer Stellung bewegt, wo zusammenwirkende Formationen des Auslösers (9) und der Feder (8) in Eingriff gebracht werden.

10. Verfahren nach einem der Ansprüche 8 oder 9, wobei die weitere Bewegung des distalen Endes der Feder (8) durch ein Sperrelement verhindert wird, das zeitweilig angrenzend an eine Lanzettnadel-Austrittsöffnung (4) in dem Gehäuse (2) angeordnet ist, durch welche die Lanzettnadel (12) vorspringt, wenn die Vorrichtung (1) ausgelöst wird.

## Revendications

1. Dispositif (1) de piqûre de la peau, comprenant:
un boîtier (2);
une aiguille de lancette (12) formée de manière intégrale avec un ressort (8) et contenue à l'intérieur du boîtier (2);
une gâchette (9) permettant de maintenir le ressort (8) dans un état comprimé jusqu'à ce que l'actionnement de la gâchette (9) par un utilisateur libère le ressort (8);
la gâchette (9) étant formée de manière intégrale avec le ressort (8); et
le boîtier (2) comportant une ouverture (5) permettant d'accéder à la gâchette (9) lorsque le ressort (8) est chargé dans le boîtier (2).

2. Dispositif (1) de piqûre de la peau selon la revendication 1, le ressort (8) étant un ressort plat plié.

3. Dispositif (1) de piqûre de la peau selon l'une quelconque des revendications précédentes, le ressort (8) et l'aiguille de lancette (12) étant formés à partir d'une simple bande de matériau.

4. Dispositif (1) de piqûre de la peau selon l'une quelconque des revendications précédentes, la gâchette (9) comprenant une plaque pour le pouce et des formations dépendantes de la plaque de pouce destinées à coopérer avec des formations sur le ressort (8) pour maintenir le ressort (8) dans un état comprimé.

5. Dispositif (1) de piqûre de la peau selon l'une quelconque des revendications précédentes, le boîtier (2) comportant une ouverture (6) permettant de charger le ressort (8) dans le boîtier (2).

6. Dispositif (1) de piqûre de la peau selon la revendication 1, la gâchette (9) s'emboîtant, lorsque le ressort (8) est chargé dans le boîtier (2), dans l'ouverture de gâchette (9) de sorte que le ressort (8) est retenu à l'intérieur du boîtier (2).

7. Dispositif (1) de piqûre de la peau selon l'une quelconque des revendications précédentes, le boîtier (2) comportant une ouverture (4) conçue pour permettre à l'aiguille de lancette (12) de sortir du boîtier (2) lorsque le ressort (8) s'allonge.

8. Procédé de fabrication d'un dispositif (1) de piqûre de la peau, le dispositif (1) de piqûre de la peau comprenant un boîtier (2) et un mécanisme de lancette (3), le mécanisme de lancette (3) comprenant une aiguille de lancette (12), un ressort (8) destiné à commander la lancette, l'aiguille de lancette (12) étant située au niveau d'une extrémité distale du ressort (8), et une gâchette (9), au moins le ressort (8) et la gâchette (9) étant formés de manière intégrale l'un avec l'autre, et le boîtier (2) comportant une ouverture (5) permettant d'accéder à la gâchette (9) lorsque le ressort (8) est chargé dans le boîtier (2), le procédé comprenant:
le chargement du mécanisme de lancette (3) dans le boîtier (2); et
l'empêchement pour l'extrémité distale du ressort (8) d'effectuer un mouvement supplémentaire tandis qu'une force est appliquée sur une extrémité proximale du ressort (8) pour comprimer le ressort (8) jusqu'à ce que la gâchette (9) s'enclenche sur le ressort (8) pour bloquer le ressort (8) dans un état comprimé.

9. Procédé selon la revendication 8, la force faisant également entrer la gâchette (9) plus avant dans le boîtier jusqu'à une position où des formations de la gâchette (9) et du ressort (8) qui coopèrent sont en prise les unes avec les autres.

10. Procédé selon l'une quelconque des revendications 8 ou 9, ledit mouvement supplémentaire de l'extrémité distale du ressort (8) étant empêché par un élément de blocage temporairement situé adjacent à l'ouverture (4) de sortie d'aiguille de lancette dans le boîtier (2), à travers laquelle l'aiguille de lancette (12) se projette lorsque le dispositif (1) est déclenché.
